(19) Europäisches Patentamt
European Patent Office
Office européen des brevets

(11) Publication number: 0 103 893 A2

(12) **EUROPEAN PATENT APPLICATION**

(21) Application number: 83109393.5

(22) Date of filing: 21.09.83

(51) Int. Cl.³: C 07 D 321/00
C 11 B 9/00

(30) Priority: 22.09.82 JP 165670/82
12.10.82 JP 178766/82
17.11.82 JP 201265/82

(43) Date of publication of application: 28.03.84 Bulletin 84/13

(84) Designated Contracting States: BE CH DE FR LI NL

(71) Applicant: DAICEL CHEMICAL INDUSTRIES, LTD.
No. 1, Teppo-cho
Sakai-shi Osaka-fu(JP)

(72) Inventor: Ueda, Yoichiro
No. 15-5, Higashi Imajuku 6-chome
Himeji-shi Hyogo(JP)

(72) Inventor: Yaso, Hayato
No. 635, Shimoyobe
Yobe-ku Himeji-shi Hyogo(JP)

(74) Representative: Hansen, Bernd, Dr.rer.nat. et al,
Hoffmann, Eitle & Partner Patentanwälte Arabellastrasse 4
D-8000 München 81(DE)

(54) **Macrocyclic diesters.**

(57) Novel 4-methyl-2,6-dioxa-1,7-dioxocycloalkane compounds represented by the formula (I)

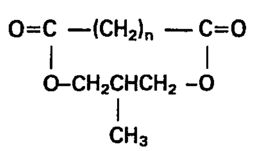

(I)

wherein n is an integer of from 8 to 11, and a perfume-imparting composition containing such novel compound, are disclosed. These compounds provide a musk-like odor.

EP 0 103 893 A2

MACROCYCLIC DIESTERS

The present invention relates to novel macrocyclic diesters having a musk odor and perfume-imparting compositions containing said macrocyclic diesters.

Besides macrocyclic ketones and macrocyclic lactones, ethylene brassylate is an important perfume ingredient and widely used in the preparation of perfumes.

Macrocyclic diesters derived using a diol component other than ethylene glycol are known. Japanese Patent Application (OPI) No. 51385/77 (corresponding to West German Patent 2,547,267 and U.S. Patents 4,157,330 and 4,105,672), for example, discloses a process for the preparation of 16 to 26-membered cyclic diesters from dodecanedioic acid and various diols with a carbon chain containing from 2 to 12 carbon atoms. The term "OPI" as used herein refers to a published unexamined Japanese patent application. It is described therein that the glycol component to be used in the foregoing process for preparation may contain one or two alkyl side chains having from 1 to 4 carbon atoms. However, no examples are described for macrocyclic diesters having one methyl side chain. Moreover, although it is described that the

final products, 16 to 26-membered cyclic diesters, can be used in the preparation of perfumes, there is no disclosure as to whether or not the compounds therein have a musk odor. I.e., such compounds although not providing their own characteristic odor, may be used as perfume ingredients, e.g., as a fixative.

Macrocyclic diesters having a methyl side chain are known. Japanese Patent Application (OPI) No. 51472/81 describes macrocyclic diesters derived from 1,2-propanediol or 1,3-butanediol and dodecanedioic acid. Japanese Patent Application (OPI) Nos. 51473/81 and 51474/81 describe similar diesters derived using brassylic acid and sebacic acid. It is described therein that these methyl side chain-containing diesters themselves do not have a noticeable odor, but when added to other perfume compositions, exhibit a perfume-improving effect.

The present inventors have succeeded in preparing novel macrocyclic diesters, i.e., 15 to 18-membered 4-methyl-2,6-dioxa-1,7-dioxocycloalkanes represented by the formula (I)

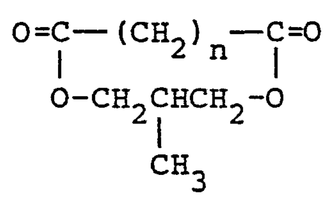

(I)

wherein n is an integer of from 8 to 11, and have further found that these macrocyclic diesters have excellent perfume characteristics.

Thus the present invention relates to: (1) 15 to 18-membered 4-methyl-2,6-dioxa-1,7-dioxocycloalkane compounds, and (2) perfume-imparting compositions containing said compounds.

Figs. 1, 3 and 5 are each an infrared absorption spectrum of 4-methyl-2,6-dioxa-1,7-dioxocycloalkane; and

Figs. 2, 4 and 6 are each a proton nuclear magnetic resonance spectrum of 4-methyl-2,6-dioxa-1,7-dioxocycloalkane.

The compounds of this invention are macrocyclic diesters having one methyl side chain and a symmetrical molecular structure. These compounds have not heretofore been known. This structural difference between the compounds of the invention and the known macrocyclic diesters having one methyl side chain at an unsymmetrical position brings about a significant difference in perfume characteristics therebetween. Moreover, the compounds of this invention are different from known methyl-substituted macrocyclic diesters in that the compounds of this invention do not have a neo-type (gem-dimethyl) structure which

is liable to provide a camphor odor and to raise the melting point.

Pure 4-methyl-2,6-dioxa-1,7-dioxocycloalkanes are colorless, transparent liquids at ordinary temperature (e.g., 20°C) and have a musk-like odor. With this respect to this musk-like odor, the compounds of the invention have surprisingly been found to quite different from known 3-methyl compounds of unsymmetrical structure (containing an asymmetric carbon atom at the 3-position which do not have any noticeable odor by themselves. The 15-membered compound (n=8 in the formula (I)) is a low melting solid since it crystallizes on standing (e.g., at 20°C) after distillation. However, because of its low rate of crystallization, it can generally be handled as a liquid.

Of the compounds of the invention, the 17-membered compound (n=10 in the formula (I)), i.e., 4-methyl-2,6-dioxa-17-dioxocycloheptadecane, is most desirable in view of its perfume characteristics and availability of precursors. The 18-membered compound (n=11 in the formula (I)), i.e., 4-methyl-2,6-dioxa-1,7-dioxocyclooctadecane, is second to the 17-membered compound in desirability.

The compounds of the invention can be prepared from known compounds. The glycol component, 2-methyl-1,3-propanediol, can be produced together with 1,4-butanediol by catalytic hydrogenation of the hydroformylated product of allyl alcohol.

The compounds of the invention can be prepared in an analogous manner as is used for the preparation of known macrocyclic diesters, for example, ethylene brassylate. That is, α,ω-dicarboxylic acid with a straight carbon chain containing from 10 to 13 carbon atoms (i.e., sebacic acid, undecanedioic acid, dodecanedioic acid, or brassylic acid) or its ester is reacted with 2-methyl-1,3-propanediol to prepare a polyester, and the polyester thus prepared is depolymerized by heating in a high vacuum to obtain 4-methyl-2,6-dioxa-1,7-dioxocycloalkane.

Thus, preparation of 4-methyl-2,6-dioxa-1,7-dioxocycloalkanes according to this invention can be carried out by appropriate selection of starting materials and reference to the method described in the aforementioned references and the method described in West German Patent 1,929,550, Japanese Patent Publications 73/72 and 29863/71, Japanese Patent Applications (OPI) 26790/73 and 28491/73. Japanese Patent Publication 25071/72, Japanese Patent Applications (OPI) 81875/80 and 69088/75, U.S. Patent 4,136,098, and Japanese Patent Applications (OPI) 84986/78, 103884/79, 120581/80, 2640/80 and 30973/81.

In preparing such polyesters, it is preferred for the reaction to be performed in the presence of a suitable esterification catalyst, such as tetrabutyl titanate. As esterification catalysts, those known for the preparation of other polyesters, such as polyethylene terephthalate, can also be used.

Catalysts containing aluminum, titanium, tin, lead, zinc, cobalt, etc., are effective for use in the depolymerization process as in the case of preparation of other macrocyclic diesters. For example, stannous oxide, stannous acetate, dibutyltin oxide, lead dioxide, and aluminum or titanium alkoxide can be used.

With respect to the depolymerization of polyester, it is generally believed that an alkyl ester derived from a primary alcohol can be more easily depolymerized than an alkyl ester derived from a secondary alcohol. Since the polyester of this invention does not contain the alkyl ester derived from a secondary alcohol, the depolymerization of the polyester can be performed in the absence of a solvent. However, if desired, an inert solvent or powder may be used in the depolymerization process in view of high efficiency of heat transfer, high distillation of crude product and ease in handling the residue in the depolymerization process and high quality of the product.

The thus-prepared cruce product contains 2-methyl-1,3-propanediol, which is used in excess, and decomposition products of dicarboxylic acid, and thus is not usually preferred to be used as such in view of its preferred use as a musk odor-providing ingredient in perfume. Hence the crude product is treated in any suitable manner, e.g., by distillation, washing water, and adsorption, and can be purified to the extent that is satisfactory.

The adsorption is effective in decolorization and removing acidic substances. For example, the crude product is diluted in a nonpolar solvent (e.g., hexane) and the resulting solution is passed through a column packed with a base-treated activated almina. The base-treated activated alumina is used in a comparatively small amount, for example, in about 10 times (by weight) the weight of the crude product to be treated.

The compounds of the invention, as described above, are colorless and have a musk-like odor in a purified state, and when used in a perfume-imparting compositions, easily mix with other conventional perfume ingredients, and are very effective in enhancing, retaining and modifying the perfume. Thus the compositions containing the compounds of the invention are more improved in perfume than those compositions not containing the compounds of the invention.

The proportion of the compound of the invention in the perfume-imparting composition is preferably from 0.5 to 30% by weight, more preferably from 0.5 to 15% by weight, particularly preferably from 1.0 to 10% by weight, although it can be varied depending on the characteristics of the composition.

The present invention is described in greater detail by reference to the following examples. It is to be noted that the examples are illustrative, and the present invention is not intended to be limited thereto.

EXAMPLE 1

Preparation of 4-methyl-2,6-dioxa-1,7-dioxocycloheptadecane

To a mixture of 200 g of dodecanedioic acid and 86.2 g of 2-methyl-1,3-propanediol was added 0.5 ml of a 6.2% by weight solution of tetrabutyl titanate in toluene, and the resulting mixture was heated with stirring. As the temperature rose, the reaction mixture became a uniform viscous liquid. When the temperature reached 155°C, water, a reaction product started to distill. The temperature was further increased. After the temperature in the system reached 195°C and almost no distillate was obtained, toluene was introduced in small portions and water was completely removed by azeotropic distillation. Then the reactor was maintained in a reduced pressure of 23 Torr by the use of an aspirator and heated at 225°C,

and materials thus distillating were removed from the system. Thus, 249.6 g of polyester was obtained which was a light brown viscous material and solidified on allowing to stand at room temperature for several days.

The polyester thus obtained was depolymerized as follows:

A mixture of 20 g of the polyester as obtained above and 1.0 g of stannous oxide was heated in a reduced pressure of 0.1 Torr. When the temperature reached 265°C, a liquid material started to distill, and this distillate was obtained until the temperature reached about 330°C.

The amount of the distillate was 10.4 g, and it was a mixture composed mainly of 2-methyl-1,3-propanediol and 4-methyl-2,6-dioxa-1,7-dioxocycloheptadecane. The desired product of 99.8% purity as determined by gas chromatographic analysis was isolated by distillation under reduced pressure of 0.1 Torr at 150-155°C (yield: 9.4 g). The product, 4-methyl-2,6-dioxa-1,7-dioxocycloheptadecane, was identified on the basis of the following physical properties:

1. Elemental Analysis

| | C | H |
|---|---|---|
| Calculated (%) | 67.57 | 9.92 |
| Found (%) | 67.39 | 10.03 |

2. Mass Spectral Analysis

   $M^+$ = 284, base peak = 114.

   Other peaks: m/e = 213, 55.

3. Infrared Spectral Analysis

   As shown in Fig. 1.

4. Proton Nuclear Magnetic Resonance Spectral Analysis

   As shown in Fig. 2.

EXAMPLE 2

Preparation of 4-methyl-2,6-dioxa-1,7-dioxocyclooctadecane

To a mixture of 70.0 g of brassylic acid and 28.4 g of 2-methyl-1,3-propanediol was added 0.5 ml of a 6.2% by weight solution of tetrabutyl titanate in toluene. Thereafter in the same manner as in Example 1, the mixture was processed to prepare a polyester. Water was distilled away at 152-196°C. After further removal of water by azeotropic distillation using toluene, a low boiling material was removed by heating in a reduced pressure of 25 Torr at 215°C. The polyester solidified when allowed to stand at room temperature. The amount of the polyester was 80.6 g.

The polyester was depolymerized in the same manner as in Example 1. A liquid material distilled at a temperature range of 230 to 330°C.

The amount of the distillate as obtained above was 12.4 g, and it was a mixture composed mainly of 2-

methyl-1,3-propanediol and 4-methyl-2,6-dioxa-1,7-dioxocyclooctadecane. The desired product of 99.8% purity as determined by gas chromatographic analysis was isolated by distillation under reduced pressure of 0.13 Torr at 160-165°C (yield: 11.2 g). The product, 4-methyl-2,6-dioxa-1,7-dioxocyclooctadecane, was identified on the basis of the following physical properties:

1. Elemental Analysis

| | C | H |
|---|---|---|
| Calculated (%) | 68.42 | 10.13 |
| Found (%) | 68.24 | 10.22 |

2. Mass Spectral Analysis

   $M^+$ = 298, base peak = 114.

   Other peaks: m/e = 227, 55.

3. Infrared Spectral Analysis

   As shown in Fig. 3.

4. Proton Nuclear Magnetic Resonance Spectral Analysis

   As shown in Fig. 4.

EXAMPLE 3

Preparation of 4-methyl-2,6-dioxa-1,7-dioxocyclopentadecane

To a mixture of 90 g of sebacic acid and 44.1 g of 2-methyl-1,3-propanediol was added 0.5 ml of a 6.2% by weight solution of tetrabutyl titanate in toluene. Thereafter in the same manner as in Example 1, the mixture was processed to prepare a polyester. Water was distilled

away at 146-196°C. After further removal of water by azeotropic distillation using toluene, a low boiling material was removed by heating in a reduced pressure at 210°C. Thus, 116 g of polyester was obtained which was a liquid of high viscosity at room temperature.

The polyester was depolymerized in the same manner as in Example 1. A liquid material distilled at a temperature range of 220 to 300°C.

The amount of the distillate as obtained above was 10.1 g, and it was a mixture composed mainly of 2-methyl-1,3-propanediol and 4-methyl-2,6-dioxa-1,7-dioxocyclopentadecane. The desired product of 97% purity as determined by gas chromatographic analysis was isolated by distillation under reduced pressure of 0.15 Torr at 149-153°C (yield: 8.5 g). The product, 4-methyl-2,6-dioxa-1,7-dioxocyclopentadecane, was identified on the basis of the following physical properties:

1. Elemental Analysis

| | C | H |
|---|---|---|
| Calculated (%) | 65.63 | 9.38 |
| Found (%) | 65.46 | 9.49 |

2. Mass Spectral Analysis

$M^+$ = 256, base peak = 185.

Other peaks: m/e = 166, 98, 55.

3. Infrared Spectral Analysis

As shown in Fig. 5.

4. Proton Nuclear Magnetic Resonance Spectral Analysis

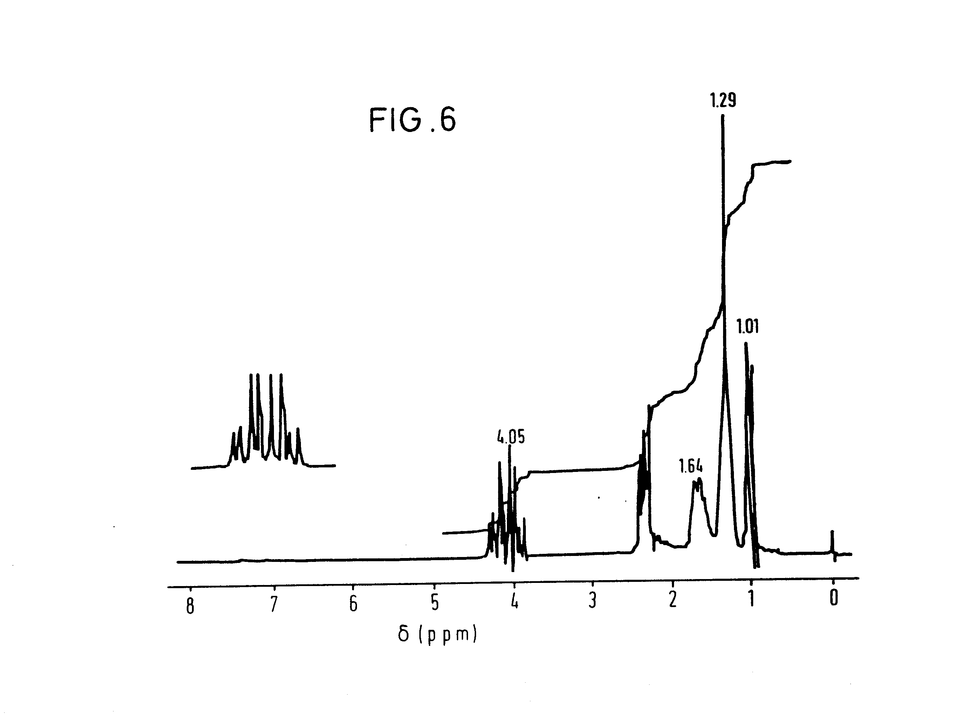

As shown in Fig. 6.

EXAMPLE 4

Using the compounds prepared in Examples 1, 2 and 3, perfume compositions (Run Nos. 1, 2 and 3) as shown in Table 1 were prepared.

These perfume-imparting compositions were aged for a sufficient period of time, and were thereafter, tested by two perfumers and compared with a control composition in which the compound of the invention was excluded. As a result, it has been judged that the perfume compositions are superior in harmony, extensibility, and retensiveness compared to the control composition.

Table 1

| Ingredient (parts by weight) | Run No. 1 | Run No. 2 | Run No. 3 |
|---|---|---|---|
| Bergamot oil | 225 | 225 | 200 |
| Oakmoss absolute | 50 | 50 | 50 |
| Patchouli oil | 20 | 20 | 20 |
| Rose absolute | 15 | 15 | 15 |
| Jasmine absolute | 20 | 20 | 20 |
| Vertiver oil | 60 | 60 | 55 |
| Sandalwood oil | 60 | 60 | 55 |
| Methyl ionone | 50 | 50 | 50 |
| Clary Sage oil | 30 | 30 | 30 |
| Coumarin | 90 | 90 | 90 |
| Labdanum absolute | 25 | 25 | 25 |
| Vanillin | 15 | 15 | 15 |
| Benzyl acetate | 25 | 25 | 25 |
| Benzoin resinoid | 50 | 50 | 50 |
| Isoeugenol | 35 | 35 | 35 |
| Cinnamyl acetate | 25 | 25 | 25 |
| Methyl salicylate | 2 | 2 | 2 |
| Angelica root oil | 25 | 25 | 25 |
| Heliotropin | 35 | 35 | 35 |
| Linalool | 30 | 30 | 30 |
| Lavender oil | 3 | 3 | 3 |
| Ylang-ylang oil | 70 | 70 | 65 |
| 4-Methyl-2,6-dioxa-1,7-dioxocycloalkane | 40 (17-membered) | 40 (18-membered | 80 (15-membered) |
| (Total amount) | (1,000) | (1,000) | (1,000) |

While the invention has been described in detail and with reference to specific embodiment thereof, it will be apparent to one skilled in the art that various changes and modifications can be made therein without departing from the spirit and scope thereof.

CLAIMS:

1. A 4-methyl-2,6-dioxa-1,7-dioxocycloalkane compound represented by the formula (I)

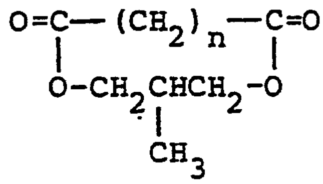

(I)

wherein n is an integer of from 8 to 11.

2. A compound as in Claim 1, wherein n is 10.

3. A compound as in Claim 1, wherein n is 11.

4. A compound as in Claim 1, wherein n is 8.

5. A perfume-imparting composition containing a 4-methyl-2,6-dioxa-1,7-dioxocycloalkane compound represented by the formula (I)

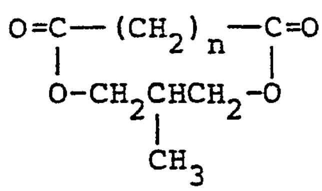

(I)

wherein n is an integer of from 8 to 11.

6. A perfume-imparting composition as in Claim 5, wherein n is 10.

7. A perfume-imparting composition as in Claim 5, wherein n is 11.

8. A perfume-imparting composition as in Claim 5, wherein n is 8.

9. A perfume-imparting composition as in Claim 5, wherein the proportion of the compound is from 0.5-30% by weight.

10. A perfume-imparting composition as in Claim 9, wherein n is 10.

11. A perfume-imparting composition as in Claim 9, wherein n is 11.

12. A perfume-imparting composition as in Claim 9, wherein n is 8.

FIG.1

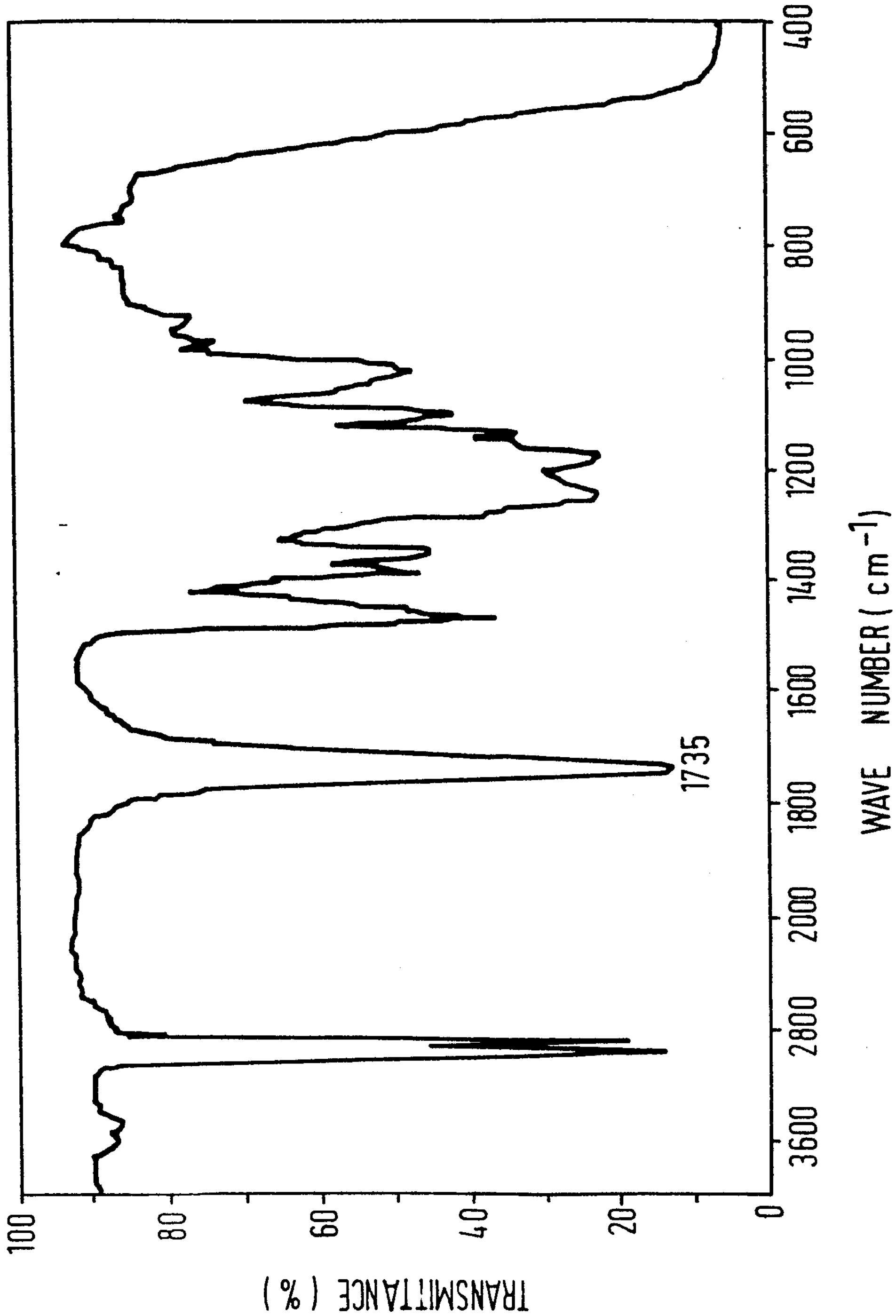

TRANSMITTANCE ( % )
100
80
60
40
20
0
WAVE NUMBER ( cm$^{-1}$)
3600
2800
2000
1800
1600
1400
1200
1000
800
600
400
1735

FIG. 2

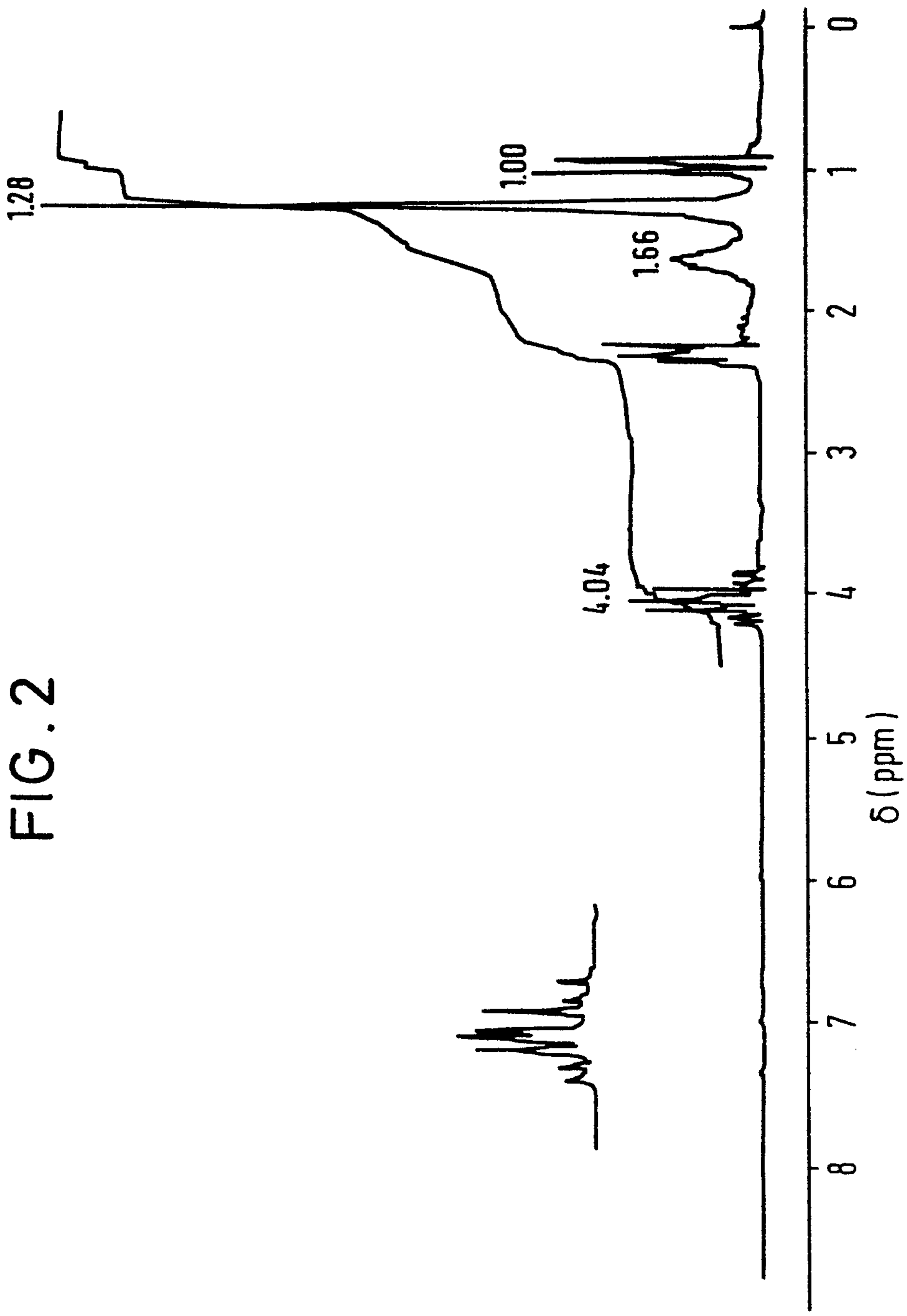

FIG.3

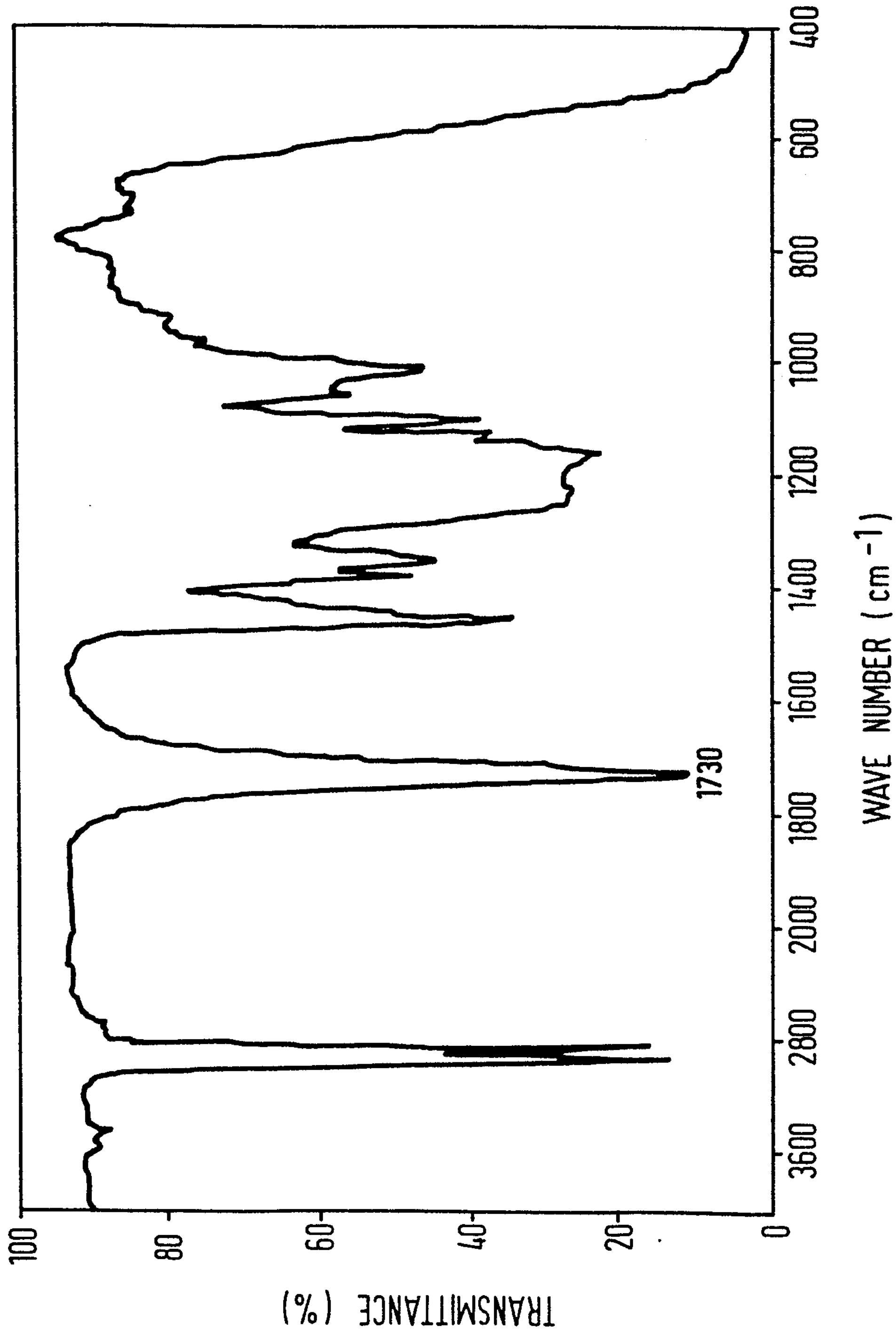
100
80
60
40
20
0
TRANSMITTANCE (%)
3600
2800
2000
1800
1600
1400
1200
1000
800
600
400
WAVE NUMBER (cm⁻¹)
1730

FIG. 4

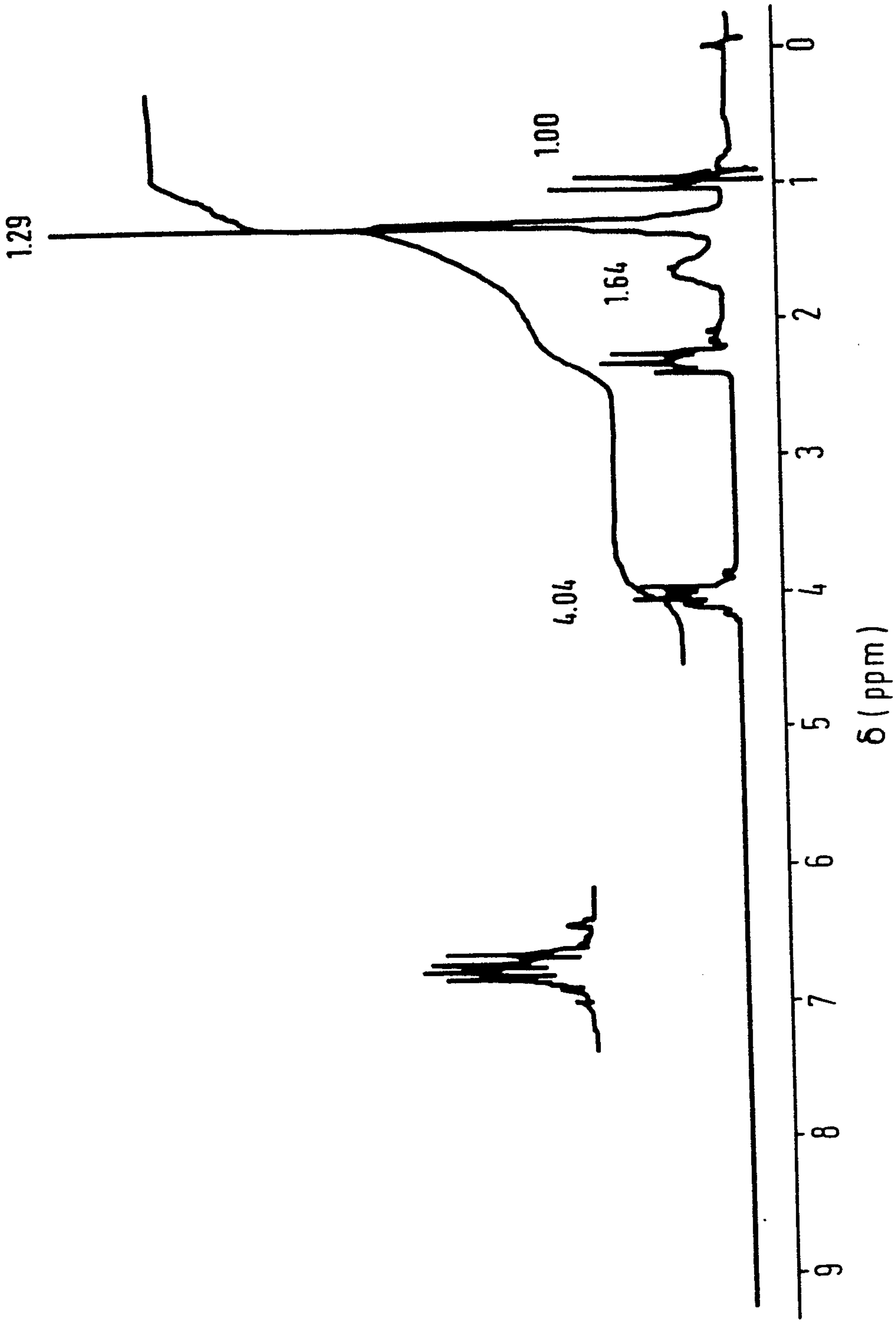

FIG.5

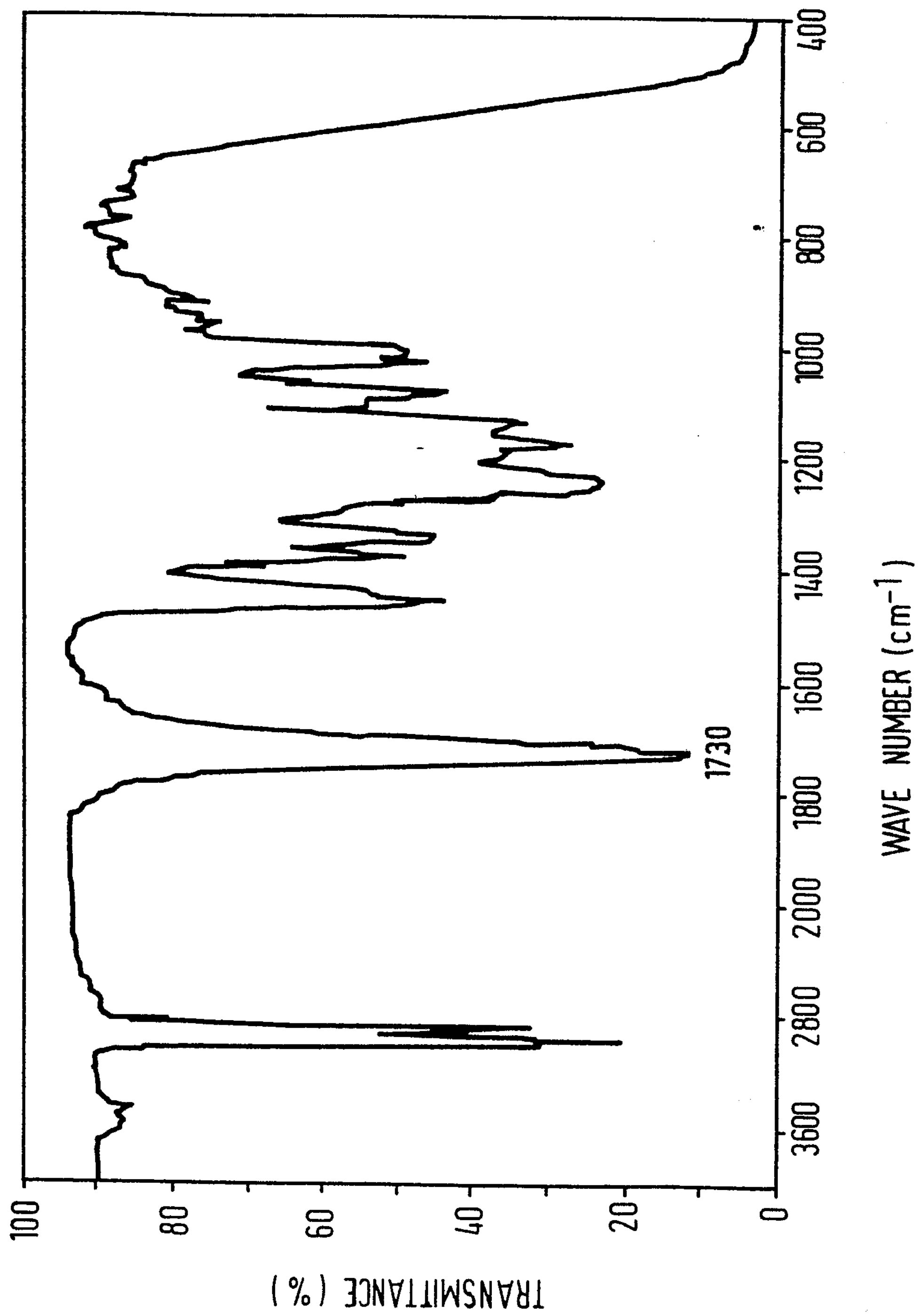
TRANSMITTANCE (%)
100
80
60
40
20
0
3600
2800
2000
1800
1600
1400
1200
1000
800
600
400
1730
WAVE NUMBER (cm$^{-1}$)

FIG. 6